# FASCICULE DE BREVET EUROPEEN

(11) **EP 2 084 538 B1**
(45) Date de publication et mention de la délivrance du brevet: **03.12.2014**
(21) Numéro de dépôt: 07848314.6
(22) Date de dépôt: 03.10.2007
(51) Int. Cl.: G01N 33/564

(54) **MÉTHODE DE DIAGNOSTIC DE TROUBLES DU COMPORTEMENT ALIMENTAIRE**
VERFAHREN ZUR DIAGNOSTIZIERUNG VON ESSSTÖRUNGEN
METHOD OF DIAGNOSING EATING DISORDERS

(30) Priorité: 03.10.2006 FR 0608664
(43) Date de publication de la demande: 05.08.2009
(73) Titulaire: Fetissov, Serguei, 76380 Montigny (FR); Dechelotte, Pierre, 76000 Rouen (FR)
(72) Inventeur: FETISSOV, Serguei, F-76380 Montigny (FR); DECHELOTTE, Pierre, F-76000 Rouen (FR); HAMZE-SINNO, Maria, F-76000 Rouen (FR); GILBERT, Danièle, F-76480 Saint Pierre de Varengeville (FR)
(74) Mandataire: Jacobson, Claude
(86) Numéro de dépôt international: PCT/FR2007/001617
(87) Numéro de publication internationale: WO 2008/040882

(56) Documents cités:
- WO-A-2005/058961
- US-B1- 6 635 479
- COMIN ROMINA ET AL: "High affinity of anti-GM(1) antibodies is associated with disease onset in experimental neuropathy" JOURNAL OF NEUROSCIENCE RESEARCH, vol. 84, no. 5, 31 juillet 2006 (2006-07-31), pages 1085-1090, XP009088528 WILEY-LISS, US ISSN: 0360-4012 cité dans la demande
- DICOU E ET AL: "Natural autoantibodies against the nerve growth factor in autoimmune diseases." JOURNAL OF NEUROIMMUNOLOGY, vol. 47, no. 2, septembre 1993 (1993-09), pages 159-167, XP009088532 ISSN: 0165-5728
- SHAW G ET AL: "alpha-MSH and neurofilament M-protein share a continuous epitope but not extended sequences. An explanation for neurofibrillary staining with alpha-MSH antibodies." FEBS LETTERS, vol. 181, no. 2, 25 février 1985 (1985-02-25), pages 343-346, XP002448115 ISSN: 0014-5793
- FETISSOV S O ET AL: "Autoantibodies against alpha-MSH, ACTH, and LHRH in anorexia and bulimia nervosa patients." PROCEEDINGS OF THE NATIONAL ACADEMY OF SCIENCES OF THE UNITED STATES OF AMERICA, vol. 99, no. 26, 24 décembre 2002 (2002-12-24), pages 17155-17160, XP009086396 ISSN: 0027-8424 cité dans la demande
- FETISSOV S O ET AL: "Aggressive behavior linked to corticotropin-reactive autoantibodies." BIOLOGICAL PSYCHIATRY, vol. 60, no. 8, 15 octobre 2006 (2006-10-15), pages 799-802, XP005697106 ISSN: 0006-3223
- FETISSOV SERGUEÏ O ET AL: "Autoantibodies against appetite-regulating peptide hormones and neuropeptides: Putative modulation by gut microflora." NUTRITION (BURBANK, LOS ANGELES COUNTY, CALIF.) APR 2008, vol. 24, no. 4, avril 2008 (2008-04), pages 348-359, XP022510832 ISSN: 0899-9007
- FETISSOV SERGUIEI O ET AL: "Autoantibodies against neuropeptides are associated with psychological traits in eating disorders", PROCEEDINGS OF THE NATIONAL ACADEMY OF SCIENCES, NATIONAL ACADEMY OF SCIENCES, US, vol. 102, no. 41, 11 October 2005 (2005-10-11), pages 14865-14870, XP009086395, ISSN: 0027-8424, DOI: 10.1073/PNAS.0507204102

## Description

### Domaine technique de la demande

La présente demande concerne le domaine du diagnostic ou du pronostic de maladies neuropsychiatriques (ci-après abrégé NP), de troubles du comportement alimentaire (ci-après abrégé TCA) ou de maladies métaboliques. Les troubles du comportement alimentaire font partie des troubles du comportement motivationnel (TCM). La présente demande se rapporte également au traitement des troubles du comportement alimentaire.

En particulier, la demande porte sur une méthode permettant de diagnostiquer ou de pronostiquer des maladies NP ou des TCA par mesure de l'affinité d'autoanticorps, d'un patient, vis-à-vis d'une molécule biologique. Les inventeurs ont découvert le rôle physiologique dans l'homéostasie et le comportement, d'autoanticorps dirigés contre certaines molécules biologiques, en particulier des neuropeptides, des hormones et des neurotransmetteurs.

On entend par « autoanticorps » (ci-après abrégé AutoAc), au sens de la présente demande, des immunoglobulines capables de se lier à des molécules qui font partie de l'organisme qui produit lesdites immunoglobulines. Ces AutoAc réagissent donc contre le « soi ». Le soi d'un individu étant défini comme l'ensemble des molécules résultant de l'expression normale de son génome.

Ces autoAc sont produites naturellement dans l'organisme, c'est-à-dire qu'ils ne sont pas administrés par une immunisation passive avec des sérums d'origine animale ou humaine ou avec des composés contenants des anticorps monoclonaux ou recombinants. Ces autoAc ne sont pas stimulés non plus par une immunisation active où l'antigène est représenté par des neuropeptides, des hormones ou leurs fragments. immunisation active où l'antigène est représenté par des neuropeptides, des hormones ou leurs fragments.

On entend par le « comportement motivationnel », tout comportement qui sert à maintenir l'homéostasie de l'organisme en le protégeant contre les agressions extérieures ou des modifications nocives des conditions environnementales. Essentiellement, le comportement motivationnel est impliqué dans la régulation des apports nutritionnels et sert également à prolonger la vie par la reproduction. Plus exactement, le comportement motivationnel inclut des comportements suivants : la prise alimentaire, la prise hydrique, le comportement de la reproduction qui inclut le comportement sexuel et maternel ou paternel, le comportement de défense ou d'agression, le comportement d'exploration, le comportement te stockage, le comportement à maintenir la température corporelle et le sommeil. Ces comportements sont régulés et intègres dans une région du cerveau appelé hypothalamus, du fait que l'altération dans le mécanisme d'un comportement affecte aussi les autres. En outre, des connexions fortes de l'hypothalamus avec des régions du cerveau responsables de la réponse émotionnelle, notamment le système limbique, implique aussi des troubles émotionnels quand un ou plusieurs mécanismes du comportement motivationnel sont altérés.

Certaines maladies NP ou TCM peuvent être caractérisées par la présence de symptômes somatiques et psychiatriques. Ceci s'explique par les interactions neuro-anatomiques mais aussi neurochimiques. On peut distinguer certaines maladies NP ou TCM dans lesquelles la composante psychiatrique est essentiellement caractérisée par des symptômes impliquant des modifications du comportement motivationnel et/ou de l'émotion.

Parmi les maladies NP ou TCM avec une forte composante psychiatrique, on peut citer notamment les TCA, l'anxiété, la dépression, la phobie sociale, la délinquance, des troubles sexuels et des troubles du sommeil.

Un groupe important de maladies NP ou TCM avec des composantes fortes somatiques et psychiatriques est formé par les TCA, où les symptômes somatiques sont associés à des troubles nutritionnels. On peut tout particulièrement citer le cas de la dénutrition dans l'anorexie mentale ou le cas de l'obésité dans les conduites boulimiques.

Dans les TCA, la composante psychiatrique peut être caractérisée par des symptômes touchant le comportement motivationnel et/ou l'émotion, tels que les troubles de la prise alimentaire et l'anxiété. Les mécanismes mis en jeu dans certaines maladies NP ou TCA peuvent également être impliqués dans la survenue de complications métaboliques, ce qui peut justifier d'inclure certaines maladies métaboliques dans le domaine de la demande, notamment l'obésité, par exemple l'obésité par excès calorique, les complications provoquées par l'obésité, le diabète de type Il et le syndrome métabolique.

### Art Antérieur

Aujourd'hui, les TCA représentent un problème important de santé publique, la prévalence de l'anorexie mentale étant estimée de 0,3% à 0,7%, celle de la boulimie à environ 3 à 5% chez les femmes, les TCA touchant les hommes 10 à 20 fois moins fréquemment. Ceci représenterait donc plus de 100,000 sujets anorexiques et plus d'un million de sujets boulimiques en France. La mortalité de l'anorexie mentale peut atteindre 10%. Il est donc clair que cette affection est extrêmement grave.

D'autre part, la persistance des symptômes pathologiques et les rechutes fréquentes observées dans toutes les formes de TCA réduisent significativement la qualité de la vie des sujets affectés.

D'autres maladies NP ou TCM que celles citées ci-dessus, sont également très répandues dans le monde. À titre d'exemple, les troubles de l'humeur et les troubles anxieux touchent ensemble plus que 60 millions de personnes en Europe (Andlin-Sobocki et al., 2005).

Jusqu'à présent, l'origine des maladies NP et TCA ou TCM restait inconnue, ou tout au moins très discutée. D'autre part, aucun marqueurs biologiques de ces maladies NP ou des TCA n'est connu, ce qui ne permet donc pas un diagnostic spécifique des maladies NP et TCA ou TCM. C'est pourquoi le diagnostic actuel des maladies NP et TCA ou TCM est purement symptomatique, ce qui n'est pas toujours exact.

Concernant le domaine thérapeutique, il n'existe pas de traitement étiologique des TCA, et des études cliniques témoignent des échecs de médicaments traditionnellement prescrits pour les maladies NP, par exemple celle portant sur l'antidépresseur fluoxetine (Walsh et al., 2006).

Malgré ces problèmes diagnostiques et thérapeutiques, la recherche expérimentale a significativement progressé dans la compréhension de la base moléculaire du comportement motivationnel et de l'émotion. Des études sur les modèles animaux ont montré le rôle important de neuropeptides et d'hormones, molécules synthétisées par les cellules neuronales et les cellules endocrines respectivement, dans la régulation physiologique du comportement motivationnel et l'émotion (de Wied, 1969). Cette recherche a permis de constater que certains neuropeptides/hormones sont impliqués préférentiellement dans des comportement motivationnel, l'émotion ou les fonctions homéostasiques (Hökfelt et al., 2003; Swaab, 2004).

Il a été montré, par exemple, que la vasopressine joue un rôle clé dans la dominance sociale, la peur et la dépression (Sewards and Sewards, 2003; Scantamburlo et al., 2005), l'ocytocine dans les relations interindividuels et la douleur, l'adrenocorticotropic hormone dans l'agression, la mémoire et la dépression (Haller et al., 2005), l'hypocrétine dans la prise alimentaire et le sommeil (De Lecea et al., 1998), et le neuropeptide Y dans la reproduction et la prise alimentaire (Pedrazzini et al., 2003).

En ce qui concerne l'homéostasie énergétique, des études ont montré que l'alpha-melanocyte-stimulating hormone (α-MSH ou α-MSH ou alpha-MSH) joue un rôle clé dans la régulation du comportement alimentaire (Fan et al., 1997; Cone, 2005) et possède un rôle anorexigène. D'autre part, l'AgRP (Agouti related protein) joue un rôle d'antagoniste de l'α-MSH par sa compétition pour le même récepteur de type mélanocortine, dont la stimulation par l'α-MSH apparaît comme la voie commune finale d'induction de la satiété (Cone, 2005). Les autres transmetteurs qui sont capables de modifier la prise alimentaire, par exemple les monoamines (Leibowitz and Alexander, 1998; Meguid et al., 2000), interagissent à plusieurs niveaux avec le système de la mélanocortine. Ainsi, cette voie finale apparaît critique pour des actions anorexigènes de la sérotonine (Heisler et al., 2002), de la leptine (Seeley et al., 1997; Marsh et al., 1999; Choi et al., 2003; Zhang and Scarpace, 2006), de l'insuline (Obici et al., 2001) et des cytokines proinflammatoires (Lawrence and Rothwell, 2001; Marks et al., 2001), mais aussi pour l'action orexigène de la ghreline via le neuropeptide Y (NPY) et l'AgRP.

Toutefois, malgré l'avancement de la recherche fondamentale, le mécanisme pathologique impliquant des neuropeptides dans la pathogenèse des maladies NP ou TCM, et en particulier des TCA, n'a pas été clarifié.

Une nouvelle hypothèse concernant le mécanisme pathologique à l'origine des maladies NP ou TCM et des TCA a récemment été proposée (Fetissov and Hökfelt, 2003). Selon cette hypothèse, les maladies NP ou TCM et les TCA pourraient être provoqués par la présence d'autoanticorps circulants dirigés contre des neuropeptides/hormones notamment impliqués dans la régulation du sommeil, mais de façon non différente de sujets contrôles (Black et al., 2005; Tanaka et al., 2006). D'autres travaux ont montré chez des sujets contrôles et des patients boulimiques la présence d'autoanticorps dirigés contre des molécules qui par leur structure chimique n'appartiennent pas aux neuropeptides mais qui sont impliquées dans la neurotransmission, telles que la sérotonine et la dopamine, ainsi que contre l'enzyme responsable de la synthèse de la noradrénaline, la dopamine β-hydroxylase (Corcos et al., 1999).

La présence de ces autoanticorps chez des patients montrée dans ces travaux pourrait suggérer un mécanisme pathologique pour des maladies NP ou TCM ou des TCA, par exemple par une neutralisation de molécules messagers. Cependant, ces autoanticorps ont également été détectés chez des sujets sains, parfois avec des titres plus élevés que chez les malades (Corcos et al., 1999; Black et al., 2005; Fetissov et al., 2005). Ainsi, la détection de tels autoanticorps pour le diagnostic des maladies NP ou TCM et TCA ne semble pas une méthode fiable. Ainsi, jusqu'à présent, l'implication réelle de ces autoanticorps dans l'étiopathogénie des maladies NP ou TCM et des TCA restait discutée.

Il existe donc un besoin pour des méthodes fiables et reproductives pour diagnostiquer et pronostiquer les maladies NP ou TCM et TCA.

### Exposé de la demande

La présente demande est basée sur la découverte que chez l'homme certaines propriétés des autoanticorps dirigés contre les neuropeptides/ hormones sont responsables de leurs actions pathogéniques et peuvent donc être utilisées pour le diagnostic, le pronostic et la thérapeutique de maladies NP, TCM, TCA et/ou métaboliques.

Les inventeurs ont découvert le rôle dans l'homéostasie et le comportement d'autoanticorps dirigés contre certaines molécules biologiques, en particulier des neuropeptides, des hormones ou des neurotransmetteurs. En particulier, les inventeurs ont mis en évidence que des maladies métaboliques, des maladies NP, TCM, des TCA, notamment l'anorexie et la boulimie, étaient liées à l'affinité et/ou l'avidité d'autoanticorps dirigés contre une molécule biologique.

On entend par « molécule biologique » au sens dans la présente démande, toute molécule produite par l'organisme humain, en particulier des neuropeptides, des hormones ou des neurotransmetteurs.

On entend par « affinité » au sens dans la présente démande, la force avec laquelle un site anticorps unique se lie à un seul épitope de l'antigène. L'affinité des anticorps se conçoit comme la résultante des forces attractives et répulsives établies entre les anticorps et les épitopes homologues de l'antigène correspondant ou avec des haptènes. L'affinité et la valence d'un anticorps peuvent être déterminées par dialyse à l'équilibre. Le calcul de l'affinité d'un site anticorps requiert l'utilisation soit d'un antigène monovalent soit d'un déterminant antigénique isolé (ou haptène).

De façon générale, les anticorps qui se combinent de façon lâche avec l'antigène et se dissocient facilement sont dits de « faible affinité» (10⁴ à 10⁵ I/mol), alors que les anticorps se fixant de façon forte sont dits de « haute affinité», (de 10⁸ à 10¹¹ l/mol). L'affinité dépend du degré de complémentarité stéréochimique entre les sites de combinaison de l'anticorps et les déterminants antigéniques.

On entend par « avidité (ou affinité fonctionnelle) » au sens dans la présente demande, la force de liaison globale d'une molécule d'anticorps pour un antigène ou une particule. Cette force peut être fonction de l'affinité paratope/épitope, de la valence de l'anticorps qui représente le nombre de site de liaisons par molécule d'anticorps (par exemple 2 pour un IgG, 10 pour un IgM) et/ou de la valence de l'antigène. L'avidité d'un anticorps pour un antigène dépend de l'affinité de chacun des sites d'anticorps pour les différents déterminants antigéniques et est supérieure à la somme de ces affinités dans le cas d'antigènes et d'anticorps multivalents, reflétant le fait que toutes les liaisons entre l'antigène et l'anticorps doivent se rompre en même temps pour que les deux molécules se séparent.

Etant donné que tout anticorps se lie a son antigène spécifique ou a plusieurs antigènes avec une certaine affinité, et étant donné que dans le cas ou l'antigène est représenté par une molécule messager comme une hormone ou un neuropeptide, l'affinité de liaison va déterminer le rôle de l'anticorps soit comme une substance bloquante de l'antigène, soit comme une substance transporteur de l'antigène. La mesure de l'affinité des autoanticorps pour les peptides impliqués dans la régulation du comportement motivationnel et de l'émotion apporte des valeur diagnostiques sur l'état fonctionnel endogène des systèmes peptidergiques correspondants.

Tandis que l'effet bloquant de l'antigène par son anticorps est bien connu, l'effet transporteur de certaines molécules messagères a été suggéré par quelques publications, comme dans Bendtzen K. et al. High-avidity autoantibodies to cytokines. Trends Immunology Today 19:5, 209-211,1998 et Dicou E. and Nerrière V., Evidence that natural autoantibodies against the nerve growth factor (NGF) may be potential carriers of NGF, Journal of Neuroimmunology, 75: 1-2, 200-203, 1997.

Un papier récent montre que le complexe de l'hormone de croissance avec son récepteur soluble possède un effet agoniste plus fort que l'hormone de croissance seule, un phénomène qui peut être appliqué aux complexes des hormones ou neuropeptides avec leurs autoAc correspondants étant donné que la formation des complexes autoAc-peptide ou récepteur-peptide implique les

Néanmoins, jusqu'à présent, la mesure de l'affinité des autoAc dirigés contre les peptides impliqués dans la régulation du comportement motivationnel, de l'humeur et de l'émotion n'a pas été rapportée. Cependant, la mesure de l'affinité des certains autoAc est proposée pour le diagnostic des maladies autoimmunes classiques ; par exemple l'affinité des autoAc contre l'insuline dans mêmes forces chimiques non covalentes, responsables de l'affinité du peptide pour le récepteur ou pour l'autoaAc.

Néanmoins, jusqu'à présent, la mesure de l'affinité des autoAc dirigés contre les peptides impliqués dans la régulation du comportement motivationnel, de l'humeur et de l'émotion n'a pas été rapportée. Cependant, la mesure de l'affinité des certains autoAc est proposée pour le diagnostic des maladies autoimmunes classiques ; par exemple l'affinité des autoAc contre l'insuline dans le diabète de type 1, comme décrit dans Schlosser M. et al, In insulin-autoantibody-positive children from the generalpopulation, antibody affinity identifies those at high and low risk. Diabetologia 48: 1830-1832, 2005.

Une autre publication montre que l'affinité des autoAc dirigés contre le ganglioside GM1, qui est par sa structure chimique un glycoshpinglipide, présent dans l'enveloppe nucléaire peut être associé avec une neuropathie de type du syndrome Guillain-Barré, comme décrit dans Comin R. et al. High affinity of anti GM1 antibodies is associated with disease onset in experimental neuropathy. J. Neurosci. Res. 84:5, 1085-90, 2006.

Le fait que la mesure de l'affinité des autoAc dirigés contre les neuropeptides/hormones impliqués dans la régulation du comportement motivationnel, de l'humeur et de l'émotion n'est pas encore utilisée peut être expliqué par la mise en évidence assez récente des autoAc contre un nombre limité de ces molécules messagères. En effet, la présence chez l'homme des autoAc a été rapportée pour l'a-MSH, la MCH, l'ACTH, la LHRH, la vasopressine et l'ocytocine par les publications récentes, comme Fetissov et al. 2002 et 2005. Et aussi pour l'hypocretine (orexine) par Black JL 2005 et Tanaka S 2006.

A part de ces peptides, les inventeurs ont récemment identifié la présence chez l'homme des autoAc dirigés contre plusieurs neuropeptides/hormones impliqués dans la régulation du comportement motivationnel, de l'humeur et de l'émotion qui à leur connaissance ne faisaient pas d'objet des publications de l'art antérieur. Ce sont des autoAc dirigés contre la leptine, la ghreline, l'AgRP (Agoutineuropeptides/hormones synthétiques.

L'invention est telle que définie par les revendications.

Un premier aspect de la présente demandedécrit un procédé de diagnostic de maladies neuropsychiatriques, de troubles du comportement alimentaire et/ou de maladies métaboliques comportant les étapes consistant à :
i) Mesurer l'affinité et/ou l'avidité d'anticorps issus d'un échantillon biologique dirigés contre une molécule biologique impliqués dans la régulation homéostasique (dans le sens physiologique), et/ou dans le comportement motivationnel, et/ou dans l'émotion;
ii) Comparer la valeur d'affinité obtenue avec une valeur contrôle.

On entend par « valeur contrôle » au sens dans la présente demande, une valeur d'affinité ou d'avidité connue qui peut être soit (i) une valeur de référence de l'affinité et/ou l'avidité d'autoanticorps chez un sujet sain ou (ii) une valeur de référence de l'affinité et/ou l'avidité d'autoanticorps chez un patient atteint d'une maladie NP, TCM, TCA ou métabolique donnée.

On entend par échantillon biologique le sang, le sérum, la salive ou tout prélèvement provenant du patient susceptible de contenir des immunoglobulines, de préférence le sang, le sérum et la salive.

La mesure des constantes d'affinité et/ou de l'avidité peut être réalisée par l'intermédiaire de méthode bien connues de l'homme du métier, et qui sont par ailleurs décrites dans le manuel de Pinckard (Pinckard, 1978).

Pour la mesure de l'affinité, on peut tout particulièrement citer la méthode de dialyse à l'équilibre. Cette méthode peut permettre de mesurer la quantité d'anticorps dans une solution. Cette méthode peut être utilisée dans le cas d'antigènes de petite taille (moins 100,000 Da), ces derniers devant pouvoir traverser la membrane à dialyse, alors que les anticorps de par leur taille ne peuvent pas diffuser.

Les deux partenaires sont placés dans des compartiments différents et l'antigène, qui peut être préalablement marqué, notamment par un isotope radioactif, est laissé à diffuser. Quand l'équilibre est atteint, l'antigène est quantifié dans chacun des deux compartiments. Par l'application de la loi d'action de masse, bien connu de l'homme du métier (Pinckard, 1978), il est possible de déterminer la constante d'association à l'équilibre.

Pour la mesure de l'avidité et de l'affinité, on peut également utiliser un test d'élution en méthode ELISA modifiée (Enzyme Linked Immunosorbent Assay), l'antigène étant en phase solide et l'anticorps en phase liquide. Un agent de dissociation, par exemple un réactif chaotropique, notamment l'isothiocyanate NH₄SCN, est utilisé pour dissocier les complexes antigènes-anticorps. Puisque les anticorps de faible avidité sont élués par des concentrations plus bases d'agent de dissociation, la concentration pour éluer 50% des anticorps liés (index d'avidité) est une bonne mesure de l'avidité moyenne des anticorps analysés (Pullen GR J. Immunol Methods, 1986, 86: 83-7).

Une méthode privilégiée pour déterminer l'affinité et/ou l'avidité d'un anticorps est basée sur l'utilisation de biocapteurs optiques permettant de mesurer les interactions moléculaires en temps réel, par analyse de résonance plasmonique de surface. La résonance des plasmons de surface est une méthode optique exploitant les ondes électromagnétiques de surface pour sonder les variations de masse, d'indice et d'épaisseur survenant à l'interface entre un métal et un diélectrique. Cette technique ne nécessite pas le marquage des biomolécules pouvant les dénaturer. de surface (RPS) exprimé en unités de résonance (RU) est en fait une mesure indirecte de la concentration en masse à la surface de la puce. Cela signifie que l'association et la dissociation du complexe anticorps/antigène peuvent être observées et qu'ultérieurement, les constantes, notamment la constante d'équilibre, peuvent être calculées.

La demande décrit tout d'abord que l'affinité et/ou l'avidité est déterminée par mesure de la résonance plasmonique de surface selon la technologie BIAcore (marque déposée) (Rich and Myszka, 2000).

L'instrument Biacore (marque déposée) a pour fonction la visualisation en temps réel des interactions entre des biomolécules non marquées, alliée à un système fluidique, permettant de faire passer sur la puce un flux de solutions diverses à débit continu. Un des réactifs, par exemple l'antigène, est retenu de manière spécifique sur une matrice appelée « sensor chip ». Les « chips » Biacore (marque déposée) les plus utilisés sont constituées d'un support de verre recouvert d'or fonctionnalisé par un biopolymère, le dextran carboxyméthylé. Les anticorps sont, eux, injectés à débit constant par un circuit microfluidique au contact de cette interface. Les changements de masse induits par l'association ou la dissociation des complexes modifient la réfringence du milieu et décalent la position de l'angle de résonance. L'enregistrement de la variation de l'angle de résonance permet de suivre en temps réel la fixation de molécules injectées sur la puce.

D'autres instruments permettant de mesurer l'affinité et/ou l'avidité d'un anticorps pour un antigène en se basant sur l'analyse de la résonance des plasmons sont commercialiés, notamment IASYS, OWLS, IBIS, TISPR-Spreeta, Interactor/SPRiLab, SPRimager II (marque déposée). On peut citer également ProteOn XPR36 (BIO-RAD).

En particulier, la molécule biologique envers laquelle l'anticorps (l'autoAc) dont on mesure l'affinité et/ou l'avidité est dirigé est (i) un neuropeptide, (ii) une hormone, ou (iii) un neurotransmetteur. L'hormone est une hormone peptidique.

La même molécule peptidique peut être appelée un neuropeptide ou une hormone en fonction de son origine ou le site d'action. Par exemple, l'α-MSH ou la ghreline secrétés par les neurones hypothalamiques sont appelés les neuropeptides, tandis que l'α-MSH secrété par l'hypophyse ou la ghreline secrété par l'estomac sont appelés hormones. En outre, l'α-MSH ou la ghreline d'origine périphérique, mais qui agissent sur les neurones peuvent être également appelé hormones ou neuropeptides.

La demande décrit que la maladie neuropsychiatrique ou le trouble du comportement alimentaire est l'anorexie mentale, et la molécule biologique est l'hormone stimulant la mélanocyte alpha (α-MSH).

La demande décrit en outre que que le trouble du comportement alimentaire est la boulimie, et la molécule biologique est l'hormone stimulant la mélanocyte alpha (α-MSH).

La demande décrit également que la maladie est un trouble du comportement alimentaire, et l'hormone est la leptine.

La demande décrit ensuite que un procédé permettant le diagnostic différentiel des TCA et des autres TCM et les neuropeptides ou les hormones envers lesquelles les autoAc dont on mesure l'affinité et/ou l'avidité sont dirigé sont: α-MSH, la leptine, la ghreline, l'AgRP (Agouti-related peptide), le NPY (Neuropeptide Y), la MCH (Melanin-concentating hormone), l'orexine (hypocretine), le PYY (Peptide YY), la galanine, l'ACTH (corticotropine), la CRH (corticotropin-releasing hormone), l'ocytocine, la vasopressine, le gonadotropine et l'hormone de croissance.

Selon un second aspect, les TCM sont les TCA tel que l'anorexie mentale, la boulimie, les TCA non spécifiées (eating disorders non otherwise specified) l'anxiété et la dépression, la phobie sociale, la délinquance, des troubles sexuels et des troubles du sommeil, la fibromyalgie, le syndrome de l'intestin irritable, l'obésité par excès calorique, les complications provoquées par l'obésité, le diabète de type II et le syndrome métabolique.

La demande décrit également que un procédé permettant de diagnostiquer des maladies neuropsychiatriques comprises dans le groupe comprenant les troubles de l'humeur, les troubles anxieux, notamment la phobie sociale, la dépression, les troubles de la mémoire par (i) la mesure de l'affinité et/ou l'avidité d'anticorps issus d'un échantillon biologique dirigés contre un neuropeptide, une hormone ou un neurotransmetteur choisi dans le groupe consistant en l'hormone libérant la corticotropine (corticotropin-releasing hormone ou CRH), l'hormone adrenocorticotropique (l'adrenocorticotropic hormone ou ACTH), la vasopressine, l'ocytocine, et les urocortines, puis (ii) la comparaison de la valeur d'affinité obtenue avec une valeur contrôle.

La demande décrit que le procédé permet de diagnostiquer des maladies neuropsychiatriques, notamment des troubles sexuels, des troubles du sommeil, des troubles anxieux, ou un trouble du comportement alimentaire par (i) la mesure de l'affinité et/ou l'avidité d'anticorps issus d'un échantillon biologique dirigés contre une molécule biologique choisie dans le groupe comprenant les peptides de la famille de la mélanocortine, le peptide AgRP (agouti-related peptide), le neuropeptide Y (NPY), l'hormone MCH (melanin-concentrating hormone), la galanine, l'hypocretine (orexine), ou la cocaïne and amphetamine-regulated transcript (CART), puis (ii) la comparaison de la valeur d'affinité obtenue avec une valeur contrôle.

La demande décrit tout d'abord que la méthode permet de diagnostiquer des maladies neuropsychiatriques associées aux douleurs somatiques ou viscérales et aux troubles anxieux chez l'homme, par (i) la mesure de l'affinité et/ou l'avidité d'anticorps issus d'un échantillon biologique dirigés contre une molécule biologique choisie dans le groupe comprenant les peptides opioïdes, les peptide tachykinines, l'ocytocine et les peptides de la famille de la mélanocortine, puis (ii) la comparaison de la valeur d'affinité obtenue avec une valeur contrôle.

La demande décrit également que la méthode permet de diagnostiquer des troubles métaboliques et/ou des troubles fonctionnels digestifs, la molécule biologique étant choisie dans le groupe comprenant la somatostatine, l'hormone GHRH (growth hormone releasing hormone), l'hormone TRH (thyrotropine releasing hormone), l'hormone GRH (gonadotropine releasing hormone), les hormones adenohypophysaires, l'insuline, le glucagon, la leptine, l'adiponectine, les cytokines, les peptides de la famille de facteurs de croissance, les peptides de la famille de facteurs trophiques, le peptide vasoactif intestinal (VIP), le pituitary adenylate cyclase activating-peptide (PACAP), le glucagon-like peptide (GLP), la ghreline, la motiline, la cholécystokinine, la sécrétine, la gastrine, l'oxyntomoduline, le peptide YY, le peptide pancréatique.

Dans la demande, le diabète de type 1 n'est pas considéré comme une maladie métabolique.

La demande décrit également un kit de diagnostic permettant de mettre en oeuvre les procédés décrit dans la demande ci-dessus. Ce kit de diagnostic contient (i) au moins une molécule biologique telle que définie précédemment, et contiendra de préférence (ii) au moins un échantillon contrôle, chaque échantillon contrôle comprenant un anticorps dirigé contre l'une desdites molécules biologiques et dont l'affinité vis-à-vis de ladite molécule biologique est connue.

Les molécules biologiques peuvent être présentes dans le kit sous formes fixées sur un support, notamment une matrice type « sensor chip » Biacore (marque déposée), constituée d'un support de verre recouvert d'or fonctionnalisé par un biopolymère, le dextran carboxyméthylé, permettant ainsi leur utilisation pour déterminer directement l'affinité et/ou l'avidité en utilisant un appareil BIAcore (marque déposée).

La demande décrit ensuite des composés modulant la concentration des complexes formés par (i) une molécule biologique impliquée dans la régulation homéostasique et/ou dans le comportement motivationnel et/ou dans l'émotion, et (ii) un anticorps X dirigé contre ladite molécule biologique, en tant que médicament.

La molécule impliquée dans la régulation homéostasique, dans le comportement motivationnel et/ou dans l'émotion peut être notamment un neuropeptide, une hormone ou un neurotransmetteur.

La demande décrit également que ledit composé module l'affinité de l'anticorps X pour ladite molécule biologique.

En outre, la demande décrit que ledit compose est une molécule « leurre » analogue à ladite molécule biologique, notamment un agoniste, capable de former un complexe avec l'anticorps X.

La demande décrit également que ledit composé est une immunoglobuline ou un fragment de celle-ci, notamment les fragments Fab et/ou Fab', qui présente une affinité pour ladite molécule biologique supérieure à celle de l'anticorps X pour la même molécule biologique, de telle sorte que l'immunoglobuline induit la dissociation de l'anticorps X impliqué dans le complexe anticorps X/molécule biologique en formant un nouveau complexe immunoglobuline/ molécule biologique. La constante d'association Ka, qui représente l'affinité, de l'immunoglobuline diffère de celle de l'anticorps X d'un facteur 10 à 10000, de préférence 100 à 1000.

La demande décrit en outre que ledit composé est une immunoglobuline ou un fragment de celle-ci, notamment les fragments Fab et/ou Fab', qui présente une affinité pour ladite molécule biologique supérieure ou inférieure à celle de l'autoAc X pour la même molécule biologique, de telle sorte que l'immunoglobuline diminue la formation des complexes autoAc X/molécule biologique en formant des nouveaux complexes immunoglobuline/ molécule biologique. La constante d'association Ka, qui représente l'affinité, de l'immunoglobuline diffère de celle de l'autoAc X d'un facteur 10 à 10000, de préférence 100 à 1000.

En particulier, l'aspect thérapeutique de cette demande concerne l'utilisation, pour le traitement des maladies NP, TCM et TCA, d'immunoglobulines dirigées contre des neuropeptides spécifiques. Par exemple, pour la thérapie de l'anorexie mentale, maladie caractérisée par la diminution de l'affinité des autoanticorps IgM pour l'α-MSH (voir la partie exemple), il serait nécessaire d'apporter aux malades des immunoglobulines, de préférence des IgM, possédant une affinité pour l'α-MSH plus forte que l'affinité des autoanticorps IgM du patient. Dans chaque cas de maladie NP, TCM, de TCA et de maladie métabolique, le choix des immunoglobulines thérapeutiques (nature de l'Ig et/ou de l'affinité) sera basé sur les résultats des tests diagnostiques décrits dans la première partie de cette demande, en mesurant l'affinité des autoanticorps dirigés contre des neuropeptides/ hormones pour déterminer quel système peptidergique est concerné.

La demande décrit ensuite qu'on peut citer le traitement de l'obésité par l'utilisation de composés modulant la concentration des complexes formés par des autoanticorps anti-ghreline et la ghreline.

En effet, un travail récent montre que l'immunisation des rats avec des fragments de ghreline diminue obésité chez le rat (Zorrilla et al., 2006). Cependant, dans ce travail, les auteurs ne considèrent pas que l'effet de l'immunisation sur le poids corporel puisse être dû aux changements des interactions entre la ghreline et les autoAc contre la ghreline. En effet, l'existence des autoAc contre la ghreline chez l'homme ou chez l'animal n'a pas été rapportée dans l'art antérieur.

Les composés décrits dans la demande peuvent être compris dans une composition comprenant en outre des excipients pharmaceutiquement acceptables ou des principes actifs admis à la pharmacopée.

La demande décrit également l'utilisation de composés modulant la concentration des complexes formés par (i) une molécule biologique impliquée dans la régulation homéostasique, et/ou dans le comportement motivationnel, et/ou dans l'émotion et (ii) un anticorps X dirigé contre ladite molécule biologique pour la fabrication de médicaments destinés à traiter des maladies neuropsychiatriques, des troubles du comportement alimentaire et/ou des maladies métaboliques. Les médicaments peuvent être destinés à traiter des TCA et des TCM.

Les maladies à traiter et les molécules biologiques décrites dans la demande sont identiques à celles détaillées ci-dessus dans la partie concernant des méthodes de diagnostiques décrits dans la demande.

Les composés décrit dans la demande peuvent être soit :
(i) des composés modulant l'affinité de l'anticorps X pour ladite molécule biologique ; soit
(ii) des molécules « leurre » analogues à ladite molécule biologique, notamment des agonistes, capable de former un complexe avec l'anticorps X ; soit
(iii) une immunoglobuline ou un fragment de celle-ci (notamment un fragment Fab et/ou un fragment Fab') qui présente une affinité pour ladite molécule biologique supérieure à celle de l'anticorps X pour la même molécule biologique, de telle sorte que l'immunoglobuline induit la dissociation de l'anticorps X impliqué dans le complexe anticorps X/molécule biologique en formant un nouveau complexe immunoglobuline/ molécule biologique ; soit
(iv) une immunoglobuline ou un fragment de celle-ci (notamment un fragment Fab et/ou un fragment Fab') qui présente une affinité pour ladite molécule biologique supérieure ou inférieure à celle de l'autoAc X pour la même molécule biologique, de telle sorte que l'immunoglobuline diminue la formation des complexes autoAc X en formant un nouveau complexe immunoglobuline/ molécule biologique.

La constante d'association Ka, qui représente l'affinité, de l'immunoglobuline diffère de celle de l'anticorps X d'un facteur pouvant aller de 10 à 10000, et notamment de 100 à 1000.

La demande décrit également l'utilisation d'immunoglobulines ou de fragments de celles-ci, dirigés spécifiquement contre des neuropeptides, des hormones ou des neurotransmetteurs, dont la constante d'affinité est connue, pour la fabrication d'un médicament destiné à traiter des maladies neuropsychiatriques, des troubles du comportement alimentaire et/ou des maladies métaboliques.

La demande divulgue l'utilisation d'immunoglobulines ou de fragments de celles-ci, notamment des IgG, IgM et/ou des IgA, ayant une affinité déterminé pour le neuropeptide α-MSH, par exemple 500 à 1500 Unité de résonance mesurée par BIAcore pour les IgM, pour la fabrication d'un médicament destiné à traiter l'anorexie mentale. Les valeurs des unités de résonance en équilibre représentent l'affinité relative des autoAc.

La demande décrit également l'utilisation d'immunoglobulines ou un fragment de celle-ci (Fab et/ou Fab') de forte affinité pour l'α-MSH (500 à 1500 RU) pour la fabrication d'un médicament destiné à traiter l'anorexie mentale.

La demande décrit également l'utilisation d'immunoglobulines ou un fragment de celle-ci (Fab et/ou Fab') avec l'affinité physiologique pour l'α-MSH (50 à 1500 RU, par exemple de 500 à 1500 RU) pour la fabrication d'un médicament destiné à traiter l'anorexie mentale. L'affinité 500 à 1500 RU est considérée comme forte. Elle convient par exemple pour les IgM. L'affinité physiologique est déterminée par les valeurs moyennes de l'affinité mesurée chez les sujets sains. Une affinité de 50 (sujets contrôles) à 200 RU (valeur sujet boulimique), est considérée comme faible par les inventeurs de la présente. Elle convient par exemple pour des IgG.

Ces valeurs d'affinité sont convertibles en unités conventionnelles par l'analyse en BIAcore de plusieurs dilutions d'un échantillon dont la concentration en autoAc est connue suivie par une transformation en Ka en utilisant par exemple le logiciel « BIAevaluation » (marque déposée) (Biacore Int. AB, Uppsala, Suède), comme décrit en détail dans l'article de Adamczyk M et al (Adamczyk et al., 2000).

La demande décrit également l'utilisation de composés modulant la concentration des complexes formés par des autoanticorps anti-ghreline et la ghreline pour la fabrication d'un médicament destiné à traiter l'obésité. On peut citer également l'utilisation de composés modulant la concentration des complexes formés par des anticorps anti-leptine et la leptine pour la fabrication d'un médicament destiné à traiter l'obésité.

Les médicaments décrits dans la demande peuvent comprendre en outre des excipients pharmaceutiques acceptables ou des principes actifs admis à la pharmacopée.

Les médicaments peuvent se présenter sous toute forme permettant une administration efficace du principe actif décrit dans la demande. Tout particulièrement ils peuvent se présenter sous forme liquide, notamment de manière à pouvoir être injectés. Ils peuvent également se présenter sous d'autres formes permettant une injection.

Ces médicaments peuvent être des solutions ou des émulsions. Tout particulièrement il s'agit d'une solution aqueuse.

Le médicament peut comprendre en outre au moins un additif, notamment choisi dans le groupe comprenant :
- un solvant, notamment aqueux ou non-aqueux, hydrophile ou hydrophobe ;
- un tensioactif, par exemple non ionique, anionique, cationique, ou un mélange de tensioactif, et tout particulièrement un tensioactif non ionique ;
- un agent chélatant ;
- un conservateur, notamment un antioxydant ou un antimicrobien ; et
- un mélange de ceux-ci.

La délivrance chez l'homme de ces composés en tant que médicament, ou des médicament comprenant ces composés, notamment des immunoglobulines d'affinité déterminée, pourra s'effectuer par administration passive (entérale ou parentérale) ou par immunisation active soit (i) avec des peptides choisis spécifiquement pour leur capacité à induire la production des immunoglobulines ayant l'affinité désirée, soit (ii) des ADN, des ARN ou leurs dérivées codant pour lesdits peptides.

L'objet de l'invention sera mieux compris à la lumière des exemples présentés ci-après donnés à titre illustratif et qui n'ont pas un caractère limitatif.

Un exemple de la présente invention est l'identification de marqueurs biologiques de TCA tels que l'anorexie mentale de type restrictive et l'anorexie mentale de type mixte avec boulimie. On parle d'anorexie restrictive lorsque le patient mange très peu alors que l'on parle d'anorexie de type mixte lorsque le patient mange très peu et a parfois des crises de boulimie suivis souvent par des vomissements.

L'affinité d'autoanticorps dirigés contre l'α-MSH provenant d'un patient atteint de TCA et d'un sujet sain, a été déterminée.

Les autoanticorps dirigés contre les neuropeptides/hormones peuvent être isolés à partir du sang ou d'autres liquides biologique humain. Tout équipement spécialement conçu pour mesurer des forces d'interactions chimiques entre les protéines, et qui permet d'exprimer des valeurs d'affinité d'une protéine pour l'autre, peut être utilisé afin de déterminer l'affinité et/ou l'avidité des autoanticorps.

### Brève description des figures

- La Figure 1 est un histogramme représentant l'affinité des autoanticorps IgG dirigés contre l'α-MSH. L'affinité est exprimée en unité de résonance (« RU ») qui est une unité arbitraire utilisé dans les méthodes de mesure d'affinité et/ou d'avidité basées sur l'étude de la résonance plasmonique.
- La Figure 2 est un histogramme représentant l'affinité des autoanticorps IgM dirigés contre l'α-MSH. L'affinité est également exprimée en unité de résonance (« RU »).

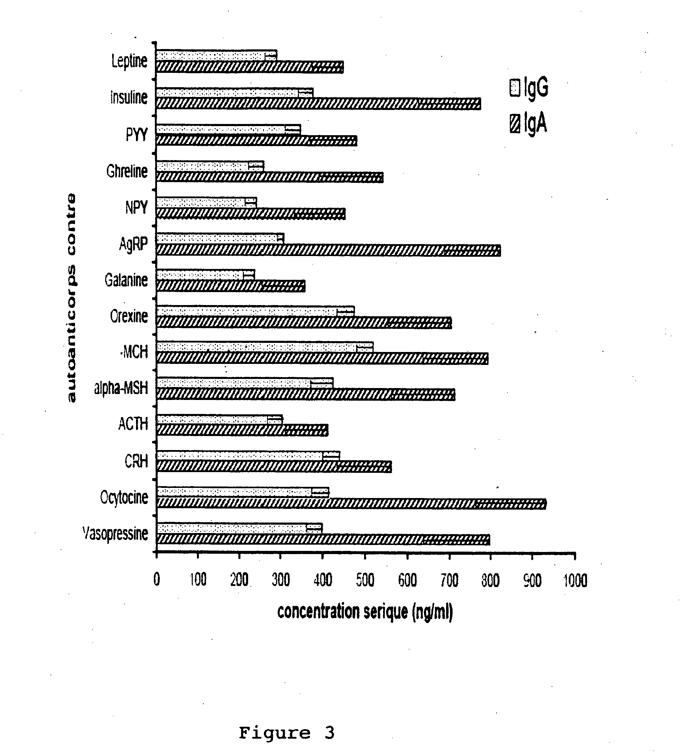

- La figure 3 est un histogramme montrant chez des sujets sains les concentrations sériques exprimée en ng/ml (moyennes ± SE) des autoAc de type IgG et IgA dirigés contre quatorze neuropeptide/hormones mesurés avec la technique ELISA directe décrite ci-dessous pour l'α-MSH.
- La figure 4 est un histogramme présentant la prise alimentaire exprimée en g (grammes) (± SE) avant injection et pendant injection d'IgG dans les expérimentations présentées dans l'exemple 2 ci-dessous.
- La figure 5 est un histogramme présentant la prise alimentaire exprimée en g (grammes) (± SE) avant injection et pendant injection intrahypothalamique d'IgG.

### EXEMPLES

### Exemple 1

Dans l'expérience décrite ci-après, la technique BIAcore a été utilisée pour déterminer l'affinité des autoanticorps.

Le protocole utilisé est le suivant :
1. Séparation de sérum par centrifugation de sang provenant d'une part de sujets atteints de TCA et, d'autre part, provenant de sujet sain (2000 g, 10 min. 4°C) ;
2. Purification des immunoglobulines G (IgG) totaux sur la protéine G / agarose à partir du sérum (Sigma St. Louis, MO, code produit P7700);
3. Dialyse des IgG totaux purifiées avec le tampon phosphate saline (PBS) à travers les membranes de dialyse (MWCO 6-8000, Spectrum lab Inc. Interchim, France) ;
4. Purification des immunoglobulines M (IgM) à partir du plasma (ou du plasma après la séparation des IgG). Le plasma est dialysé à travers les membranes de dialyse (MWCO 6-8000, Spectrum lab Inc. Interchim, France) contre 1 litre d'eau distillée. Après l'apparition du précipitât dans les membranes, le contenu est centrifugé et le culot contenant les IgM est dilué dans un tampon phosphate, NaCl 0,2M;
5. Préparation de la matrice (Sensor chip CM5, Biacore Int. AB, Uppsala, Suède) par la liaison avec 35 µl du peptide α-MSH (Bachem, Suisse, code produit H-1075) dilué à 1 mg/ml dans un tampon sodium acétate 10mM en utilisant un Kit pour le Couplage des Amines (Biacore Int. AB, Uppsala, Suède, Code produit: BR-1000-50) qui permet l'immobilisation covalente des ligands portant un groupe primaire amine.
6. Mesure de l'affinité des autoanticorps lier à α-MSH par l'injection dans un appareil Biacore (Biacore Int. AB, Uppsala, Suède) de 25 µl de chaque échantillon d'IgG totaux et d'IgM purifiés.
7. Analyse des résultats par la comparaison de valeurs de l'affinité ('RU'-unité de résonance) entre les sujets atteints de TCA et les sujets sains (contrôle).

Le même protocole est valable pour les mesures de l'affinité de toutes les classes d'Ig, notamment IgA, IgE, IgG et IgM, dirigés contre d'autres neuropeptides/hormones et dans les cas d'autre maladies NP, TCM, TCA ou métaboliques.

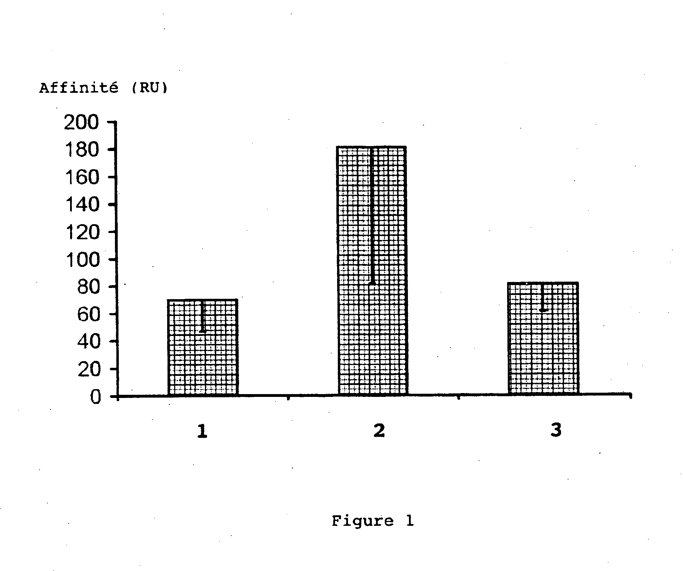

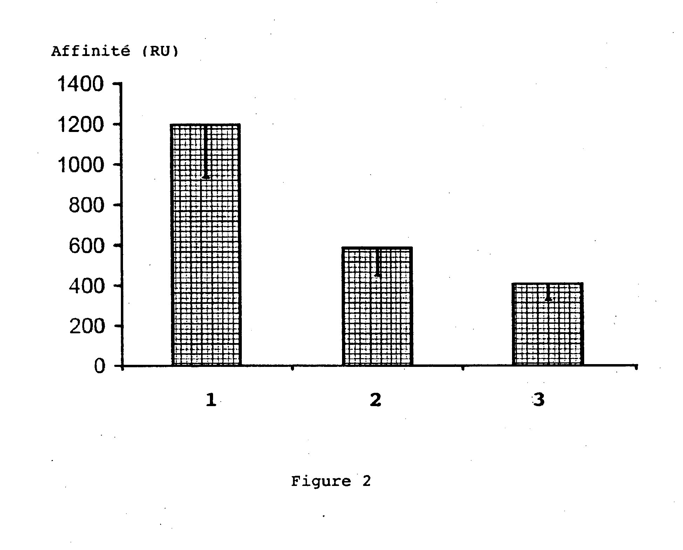

Les résultats sont représentés aux Figures 1 et 2.

La Figure 1 est un histogramme représentant l'affinité des autoanticorps IgG dirigés contre l'α-MSH. La colonne (1) représente les sujets sains, la colonne (2) représente les sujets souffrant d'anorexie mixte et la colonne (3) représente les sujets souffrant d'anorexie restrictive.

La Figure 2 est un histogramme représentant l'affinité des autoanticorps IgM dirigés contre l'α-MSH. La colonne (1) représente les sujets sains, la colonne (2) représente les sujets souffrant d'anorexie mixte et la colonne (3) représente les sujets souffrant d'anorexie restrictive.L'affinité est exprimée en unité de résonance (« RU ») qui est une unité arbitraire utilisé dans les méthodes de mesure d'affinité et/ou d'avidité basées sur l'étude de la résonance plasmonique.

L'analyse de la Figure 1 montre que l'augmentation de l'affinité des autoanticorps IgG dirigés contre l'α-MSH est significativement associée à l'anorexie de type mixte. En effet, l'affinité des IgG anti-α-MSH présents chez les patients souffrant d'anorexie mixte (colonne(2)) est significativement supérieure à celle des autoanticorps IgG anti-α-MSH présents chez les sujets sains (colonne (1)) et des patients souffrant d'anorexie restrictive (colonne (3)). Par ailleurs, l'affinité des autoanticorps IgG anti-α-MSH est similaire entre un sujet sain et un sujet atteint d'anorexie restrictive (N=10, Kruskal-Wallis test p<0.01.* Dunn's post-hoc test p<0.05).

L'analyse de la Figure 2 montre que la diminution de l'affinité des autoanticorps IgM dirigés contre l'α-MSH est significativement associée avec l'anorexie de type mixte et de type restrictive (N=10, ANOVA p<0.05. * Tukey-Kramer post-hoc test p<0.05, a* T-test p<0.05). L'affinité les autoanticorps IgM des patients souffrant d'anorexie mixte ou d'anorexie restrictive (respectivement colonne (2) et (3)) est significativement inférieure à celle des autoanticorps IgM présents chez les sujets sains (colonne (1)).

La méthode selon l'invention permet donc de déterminer que l'augmentation de l'affinité des autoanticorps de type IgG (total) est significativement associée avec la boulimie chez les patients présentant une anorexie de type mixte (Figure 1).

Sans vouloir être lié par une explication théorique, l'augmentation de l'affinité pourrait entraîner la crise boulimique par la neutralisation rapide de l'a-MSH sécrétée au cours d'un repas, l'α-MSH ne serait donc plus capable d'assurer son action physiologique d'induction de la satiété et d'arrêt de la prise alimentaire.

En outre, la méthode selon l'invention permet de déterminer que la diminution de l'affinité des autoanticorps de type IgM dirigés contre l'α-MSH est significativement associée avec les périodes anorexiques chez des patients avec anorexie de type restrictif ou type mixte (Figure 2).

Cette diminution pourrait être responsable du défaut de l'élimination de l'α-MSH de la circulation où ce peptide anorexigène est normalement sécrété de façon phasique, avec pour conséquence la perception permanente de satiété chez les sujets anorexiques.

La méthode selon l'invention a permis de déterminer qu'après extraction des IgG et IgM, l'affinité résiduelle du plasma pour l'α-MSH est significativement élevée chez les patientes souffrant d'anorexie de type mixte. Ce changement de l'affinité résiduelle est similaire au changement d'affinité observé pour les IgG.

Ainsi, d'autres classes d'immunoglobulines possèdent des valeurs diagnostiques pour les maladies NP et TCA séparément ou en conjonction avec les valeurs d'affinité des IgG et/ou des IgM. Notamment, les IgA dirigées contre l'α-MSH ou contre d'autres neuropeptides/hormones sont présentes chez les sujets sains et chez les sujets atteints de TCA et ont une valeur diagnostique.

D'autre part, l'affinité des autoanticorps (autoAc) est également responsable de la formation de complexes immuns, elle peut indirectement influencer l'équilibre entre le niveau d'autoanticorps libres et le niveau d'autoanticorps liés à l'antigène.

La présence des complexes immuns entre autoAc et neuropeptide/ hormone va influencer l'activité de ce neuropeptide/hormone auprès de son récepteur spécifique.

La présence des complexes immuns dans un échantillon biologique va limiter ou rendre impossible la mesure de l'affinité par la technique de la résonance plasmonique de surface pour un neuropeptide/ hormone d'une certaine quantité d'autoAc qui sont déjà fortement liés avec son antigène dans un complexe immun.

La présence des complexes immuns entre autoAc et neuropeptide/hormone dans un échantillon biologique peut être déterminée par la mesure du ratio entre les autoAc libres et liés (détermination du ratio autoanticorps dirigés contre les neuropeptides / hormones libre par rapport aux autoanticorps liés) comme suit :
(i) réalisation d'un ELISA avec dissociation de l'antigène;
(ii) réalisation d'un ELISA directe ;
(iii) comparaison des résultats obtenus aux étapes (i) et (ii) et détermination du pourcentage des autoAc qui existent sous forme de complexes immuns.

Cette méthode a permis de déterminer que des autoAc de type IgG (total) capable de se lier à l'α-MSH ou à l'hormone de croissance existent en complexes immuns à des niveaux augmentés chez les patientes souffrant d'anorexie mentale de type restrictive. La même méthode a permis de déterminer que des autoAc de type IgM qui peuvent se lier avec l'α-MSH forment de complexes immuns à des niveaux réduits chez les patients souffrant d'anorexie de type restrictive ou de type mixte.

Pour l'expérience rapportée ci-dessus, la technique ELISA a été utilisée pour déterminer les niveaux des autoAc libres ou totaux qui peuvent se lier avec l'α-MSH.

Un protocole pouvant être utilisé pour déterminer le pourcentage d'autoanticorps présents sous forme de complexes immuns est le suivant :
1. Le peptide α-MSH (Bachem, Suisse, code produit H-1075) a été dilué à une concentration de 2 µg/ml dans un tampon NaHCO₃ 100 mM pH 9.6 et couplé (100 µl par puit) sur microplaques de 96 puits, Maxisorp (Nunc, Rochester, NY, Etat Unis) pendant 24h à 4°C.
2. Après 3 lavages de 5 min avec une solution de PBS à 0.05% Tween 200, pH 7.4, les plaques ont été incubées pendant la nuit avec les échantillons de plasma de patient dilué (i) dans un tampon PBS (1 :100) pour réaliser un ELISA direct ou (ii) dans un tampon NaCl, 3M Glycine 1.5M, pH 8.9 (1 :200-1 :1000) pour réaliser un ELISA avec dissociation des complexes immuns.
3. Après 3 lavages de 5 min avec une solution de PBS à 0.05% Tween 200, pH 7.4, les plaques ont été incubées avec les anticorps de lapin (1:2,000) dirigés contre les IgG humaines conjuguées à la phosphatase alcaline (Jackson ImmunoResearch Laboratories Inc., West Grove, PA) pendant 3 heurs à température ambiante.
4. Après 3 lavages de 5 min avec une solution de PBS à 0.05% Tween 200, pH 7.4, les plaques ont été incubées avec 100 µl de la solution de p-nitrophenyl phosphate (Sigma, N2770) pendant 40 min à température ambiante.
5. La réaction est arrêtée par 50 ml de NaOH 3N. La densité optique est déterminée à 405 nm dans un lecteur de microplaques.
6. En divisant les valeurs de densité optique obtenues en ELISA directe par la valeur de densité optique obtenue en ELISA avec dissociation, un ratio anticorps libres/anticorps complexés est déterminé. Plus le ratio est élevé, moins de complexes immuns sont présents.

Ainsi, les variations d'affinité ou d'avidité (qui représente l'affinité totale) des autoanticorps (notamment IgG, IgM et/ou IgA) dirigés contre l'α-MSH ont été identifiées comme des marqueurs biologiques des TCA. L'utilisation de ces marqueurs dans le diagnostic des TCA est justifiée par le rôle important de l'α-MSH dans la régulation de l'appétit.

Cette utilisation est également jusitifée par les résultats d'un modèle animal du transfert passif des autoAc contre α-MSH présentés ci-dessous.

Même si l'affinité des autoanticorps dirigés contre l'α-MSH reste le marqueur clé des TCA, il est cependant clair que, du fait du caractère multifactoriel des TCA, ceux-ci peuvent être associés à une dérégulation d'autres systèmes peptidergiques que l'α-MSH de façon variable selon les patients.

En appliquant le même principe, des changements d'affinité des autoanticorps dirigés contre d'autres molécules biologiques, notamment des neuropeptides/ hormones, seront identifiés comme marqueurs biologiques d'autres maladies NP en fonction du rôle majeur de chaque neuropeptide/ hormone dans les mécanismes homéostatiques, du comportement motivationnel, de l'émotion ou du métabolisme. Citons, par exemple, le changement de l'affinité des autoanticorps dirigés contre la vasopressine l'ocytocine et de l'ACTH comme marqueurs de la dépression ou de la délinquance.

La signification biologique des changements de l'affinité des autoanticorps dirigés contre chaque neuropeptide/ hormone individuel, ou en combinaison, dans les maladies NP, TCA et maladies métaboliques peut donc être déterminée en appliquant les méthodes de diagnostic selon la présente invention.

Dans la phase initiale de mise au point, des tests diagnostiques des maladies NP et TCA déterminant les valeurs d'affinité serviront comme marqueurs pathologiques, par comparaison avec les valeurs d'affinité correspondantes déterminées chez des sujets sains.

La détection des marqueurs biologiques selon la présente invention permettra le diagnostic rapide, spécifique et non équivoque des maladies NP, TCA et métabolique chez les sujets nécessitant une prise en charge spécialisée, facilitant l'utilisation d'une approche thérapeutique étiopathogénique et individuellement ciblée pour chaque patient.

En outre, la détection de ces marqueurs au cours du suivi de ces maladies NP, TCA, ou métaboliques contribuera à l'évaluation biologique de l'efficacité thérapeutique des traitements et de l'évolution vers la guérison.

Enfin, la détection de ces marqueurs chez des individus qui présentent des symptômes débutants de maladies NP, TCM ou TCA ou chez des individus issus de familles atteintes de maladies NP, TCM ou de TCA pourra contribuer à un repérage et une prise en charge précoce de ces affections, voire à la prévention de leur développement.

### Exemple 2

Pour justifier le nouveau concept diagnostique des TCA par la mesure de l'affinité des autoAc pour neuropeptide /hormones impliqués dans la régulation du comportement motivationnel, les inventeurs ont développé un modèle expérimental du transfert passif chez le rat des autoAc contre α-MSH avec une forte ou faible affinité.

Les autoAc de type IgG ont été purifiés du sérum des sujets atteints de l'anorexie mentale ou de la boulimie, l'affinité de ces IgG pour α-MSH a été mesurée avec la technique de résonance plasmonique de surface (BiaCore) décrite en détail dans la présente en montrant les valeurs significativement plus élevées chez les sujets boulimiques (voir Figure 2 annexée).

Les IgG provenant de sujets anorexiques ont été réunis dans un seul pool A, tandis que les IgG de provenance de sujets boulimiques ont été réunis dans un autre pool B. Les autoAc contre α-MSH ont été purifiés du pool A et du pool B par l'affinité contre le peptide α-MSH sur deux colonnes chromatographiques.

La présence des IgG contre l'α-MSH dans l'éluat a été confirmée par l'ELISA. Les IgG contre α-MSH ont été lyophilisés et dilués dans un tampon ressemblant le liquide céphalo-rachidien avec la concentration finale de 3 mg /ml.

Les rats Wistar ont été anesthésiés et implantés en utilisant un appareil stéréotaxique avec des canules métalliques (PlasticOne) visant la partie ventro-basale de l'hypothalamus unilatérale. Une semaine après l'implantation de canules, les rats (n=30) ont été divisés en 3 groupes présentant un poids corporel égal entre les groups.

Les rats ont été mis à un régime restrictif de nourriture qui a été accessible entre 10h00 et 18h00 tous les jours et de l'eau a été donnée ad libitum. Trente minute avant la provision de la nourriture les rats vigiles ont reçu des injections dans l'hypothalamus par la canule de 1 microlitre par jour pendant cinq jours:
- premier groupe de rats a reçu des IgG purifiés du pool A,

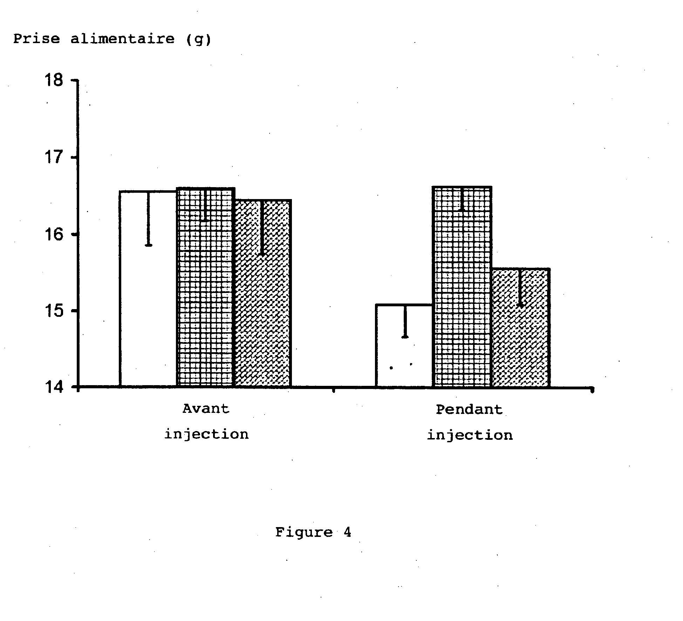

La prise alimentaire et le poids corporel ont été mesurés tous les jours. Les inventeurs ont observé que les rats injectés par des IgG du pool B ont mangé significativement d'avantage par rapport aux rats injectés par des IgG de pool A, comme représenté sur la figure 4 annexée. Sur cette figure est représentée la prise alimentaire (g±SE) par jour pendant la restriction :
- les trois colonnes de gauche (dans l'ordre, de gauche à droite), Pool A, Pool B et tampon seul, correspondant, avant injection intrahypothalamique ;
- les trois colonnes de droite, les mêmes groupes pendant l'injection.

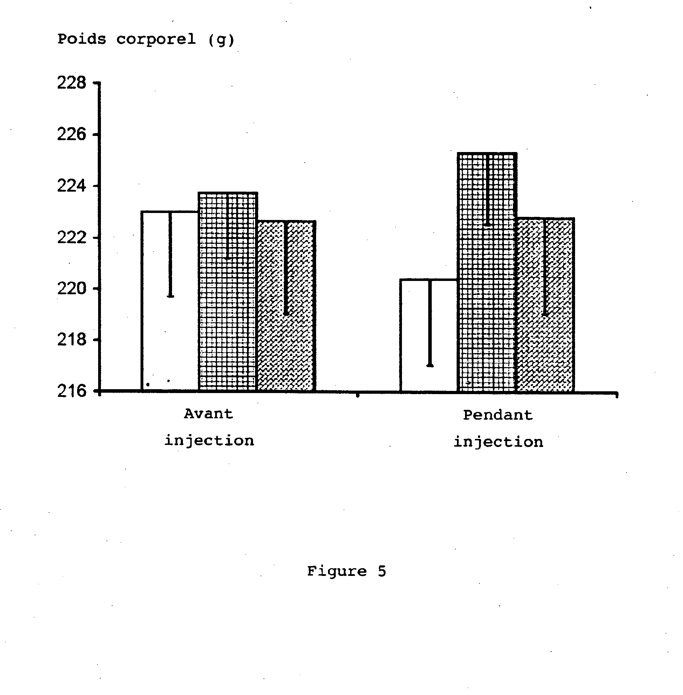

Kruskal-Wallis test entre groupes pendant injection, p=0.02, Dunn's test Pool A vs. Pool B p<0.05. En outre, les rats injectés par des IgG du pool B ont gagné du poids corporel, tandis que les rats injectes par des IgG de pool A ont perdu du poids corporel par rapport à période avant les injections, comme représenté sur la figure 5 annexée. Sur cette figure 5 sont réprésentées :
- la prise de poids corporel (g±SE) pendant la restriction, respectivement les trois colonnes de gauche Pool A, Pool B et tampon seul, correspondant, avant injection intrahypothalamique ;
- les trois colonnes de droite les mêmes groupes pendant l'injection. T-test apparié : Pool A avant vs. Pool A pendant injection, p<0.01, Pool B avant vs. Pendant injection p<0.05.

Cette expérimentation a montré :
i) l'importance fonctionnelle causal de l'affinité d'un autoAc contre un neuropeptide /hormone impliqué dans la régulation du comportement motivationnel tels que l'α-MSH;
ii) la possibilité de modifier la prise alimentaire par le changement de la concentration d'autoAc contre l'α-MSH.

Il faut néanmoins souligner que l'injection intracérébrale des autoAc utilisée dans cette expérimentation doit être considérée comme un modèle expérimental expliquant des mécanismes d'action de ces autoAc sur le comportement alimentaire mais pas comme une voie thérapeutique qui doit préférentiellement utiliser une des routes périphériques.

### LISTE DES RÉFÉRENCES

1. Adamczyk M, Moore JA, Yu Z. 2000. Application of Surface Plasmon Resonance toward Studies of Low-Molecular-Weight Antigen-Antibody Binding Interactions. Methods 20:319-328.
2. Andlin-Sobocki P, Jonsson B, Wittchen HU, Olesen J. 2005. Cost of disorders of the brain in Europe. Eur J Neurol 12 Suppl 1:1-27.
3. Bendtzen K. et al., High-avidity autoantibodies to cytokines. Trends Immunology Today 19:5, 209-211,1998.
4. Black JL, 3rd, Silber MH, Krahn LE, Fredrickson PA, Pankratz VS, Avula R, Walker DL, Slocumb NL. 2005. Analysis of hypocretin (orexin) antibodies in patients with narcolepsy. Sleep 28:427-431.
5. Choi YH, Li C, Page K, Westby A, Della-Fera MA, Lin J, Hartzell DL, Baile CA. 2003. Melanocortin receptors mediate leptin effects on feeding and body weight but not adipose apoptosis. Physiol Behav 79:795-801.
6. Cone RD. 2005. Anatomy and regulation of the central melanocortin system. Nat Neurosci 8:571-578.
7. Comin R. et al. High affinity of anti GM1 antibodies is associated with disease onset in experimental neuropathy. J. Neurosci. Res. 84:5, 1085-90, 2006.
8. Corcos M, Atger F, Levy-Soussan P, Avrameas S, Guilbert B, Cayol V, Jeammet P. 1999. Bulimia nervosa and autoimmunity. Psychiatry Res 87:77-82.
9. De Lecea L, Kilduff TS, Peyron C, Gao X, Foye PE, Danielson PE, Fukuhara C, Battenberg EL, Gautvik VT, Bartlett FS, 2nd, Frankel WN, van den Pol AN, Bloom FE, Gautvik KM, Sutcliffe JG. 1998. The hypocretins: hypothalamus-specific peptides with neuroexcitatory activity. Proc Natl Acad Sci U S A 95:322-327.
10. de Wied D. 1969. Effects of peptide hormones on behavior. In: Ganong WF, Martini L, Editors. Frontiers in Neuroendocrinology. New York: Oxford University Press. p 97-140.
11. Dicou E. and Nerrière V., Evidence that natural autoantibodies against the nerve growth factor (NGF) may be potential carriers of NGF. Journal of Neuroimmunology, 75: 1-2, 200-203, 1997.
12. Fan W, Boston BA, Kesterson RA, Hruby VJ, Cone RD. 1997. Role of melanocortinergic neurons in feeding and the agouti obesity syndrome. Nature 385:165-168.
13. Fetissov SO, Hallman J, Oreland L, af Klinteberg B, Grenbäck E, Hulting AL, Hökfelt T. 2002. Autoantibodies against a-MSH, ACTH, and LHRH in anorexia and bulimia nervosa patients. Proc Natl Acad Sci USA 99:17155-17160.
14. Fetissov SO, Harro J, Jaanisk M, Järv A, Podar I, Allik J, Nilsson I, Sakthivel P, Lefvert AK, Hökfelt T. 2005. Autoantibodies against neuropeptides are associated with psychological traits in eating disorders. Proc Natl Acad Sci USA 102:14865-14870.
15. Fetissov SO, Hökfelt T. 2003. Autoimmune component in anorexia and bulimia nervosa. A new hypothesis for eating disorders. In: SSIB 11th Annual Meeting. Groningen, The Netherlands.
16. Haller J, Mikics E, Halasz J, Toth M. 2005. Mechanisms differentiating normal from abnormal aggression: glucocorticoids and serotonin. Eur J Pharmacol 526:89-100.
17. Heisler LK, Cowley MA, Tecott LH, Fan W, Low MJ, Smart JL, Rubinstein M, Tatro JB, Marcus JN, Holstege H, Lee CE, Cone RD, Elmquist JK. 2002. Activation of Central Melanocortin Pathways by Fenfluramine. Science 297:609-611.
18. Hökfelt T, Bartfai T, Bloom F. 2003. Neuropeptides: opportunities for drug discovery. Lancet Neurol 2:463-472.
19. Lawrence CB, Rothwell NJ. 2001. Anorexic But Not Pyrogenic Actions of Interleukin-1 are Modulated by Central Melanocortin-3/4 Receptors in the Rat. Journal of Neuroendocrinology 13:490-495.
20. Leibowitz SF, Alexander JT. 1998. Hypothalamic serotonin in control of eating behavior, meal size, and body weight. Biological Psychiatry 44:851-864.
21. Marks DL, Ling N, Cone RD. 2001. Role of the central melanocortin system in cachexia. Cancer Res 61:1432-1438.
22. Marsh DJ, Hollopeter G, Huszar D, Laufer R, Yagaloff KA, Fisher SL, Burn P, Palmiter RD. 1999. Response of melanocortin-4 receptor-deficient mice to anorectic and orexigenic peptides. Nat Genet 21:119-122.
23. Meguid MM, Fetissov SO, Varma M, Sato T, Zhang L, Laviano A, Rossi-Fanelli F. 2000. Hypothalamic dopamine and serotonin in the regulation of food intake. Nutrition 16:843-857.
24. Obici S, Feng Z, Tan J, Liu L, Karkanias G, Rossetti L. 2001. Central melanocortin receptors regulate insulin action. J. Clin. Invest. 108:1079-1085.
25. Pedrazzini T, Pralong F, Grouzmann E. 2003. Neuropeptide Y: the universal soldier. Cell Mol Life Sci 60:350-377.
26. Pinckard RN. 1978. Equilibrium dialysis and préparation of hapten conjugates. In: Weir DM, Editor. Handbook of experimental immunology. Oxford: Blackwell Science. p 17.11-17.23.
27. Rich RL, Myszka DG. 2000. Advances in surface plasmon résonance biosensor analysis. Current Opinion in Biotechnology 11:54-61.
28. Scantamburlo G, Hansenne M, Fuchs S, Pitchot W, Pinto E, Reggers J, Ansseau M, Legros JJ. 2005. AVP- and OT-neurophysins response to apomorphine and clonidine in major depression. Psychoneuroendocrinology 30:839-845.
29. Seeley RJ, Yagaloff KA, Fisher SL, Bum P, Thiele TE, van Dijk G, Baskin DG, Schwartz MW. 1997. Melanocortin receptors in leptin effects. Nature 390:349.
30. Sewards TV, Sewards MA. 2003. Fear and power-dominance motivation: proposed contributions of peptide hormones present in cerebrospinal fluid and plasma. Neuroscience & Biobehavioral Reviews 27:247-267.
31. Schlosser M. et al, In insulin-autoantibody-positive children from the generalpopulation, antibody affinity identifies those at high and low risk. Diabetologia 48: 1830-1832, 2005.
32. Swaab DF. 2004. Neuropeptides in hypothalamic neuronal disorders. Int Rev Cytol 240:305-375.
33. Tanaka S, Honda Y, Inoue Y, Honda M. 2006. Détection of autoantibodies against hypocretin, hcrtrl, and hcrtr2 in narcolepsy: anti-Hcrt system antibody in narcolepsy. Sleep 29:633-638.
34. Walsh BT, Kaplan AS, Attia E, Olmsted M, Parides M, Carter JC, Pike KM, Devlin MJ, Woodside B, Roberto CA, Rockert W. 2006. Fluoxetine after weight restoration in anorexia nervosa: a randomized controlled trial. Jama 295:2605-2612.
35. Zhang Y, Scarpace PJ. 2006. Circumventing central leptin résistance: Lessons from central leptin and POMC gene delivery. Peptides 27:350-364.
36. Zorrilla EP, Iwasaki S, Moss JA, Chang J, Otsuji J, Inoue K, Meijler MM, Janda KD. 2006. Vaccination against weight gain. Proc Natl Acad Sci USA. 29:12961-2.

## Revendications

1. Procédé de diagnostic de l'anorexie mentale de type restrictive et l'anorexie mentale de type mixte avec boulimie, ledit procédé comportant les étapes consistant à :
i) Mesurer l'affinité d'anticorps issus d'un échantillon biologique d'un patient dirigés contre alpha-MSH (« alpha melanocyte-stimulating hormone ») (α-MSH);
ii) Comparer la valeur d'affinité obtenue avec une valeur de référence de l'affinité d'autoanticorps dirigés contre l'α-MSH chez un sujet sain, et
conclure que le patient souffre d'anorexie mentale de type mixte avec boulimie ou l'anorexie mentale de type restrictive si l'affinité des autoanticorps IgM dirigés contre l'α-MSH mesurée à l'étape a) est significativement inférieure à celle des autoanticorps IgM dirigés contre l'α-MSH présents chez les sujets sains, ou
le patient présente une anorexie mentale de type mixte avec boulimie si l'affinité des autoanticorps IgG dirigés contre l'α-MSH mesurée à l'étape a) est significativement augmentée par rapport à la valeur de référence de l'affinité d'autoanticorps IgG dirigés contre l'α-MSH chez un sujet sain, ou
le patient présente une anorexie mentale de type restrictive quand l'affinité d'autoanticorps IgM dirigés contre l'α-MSH mesurée à l'étape a) est significativement inférieure à celle des autoanticorps IgM dirigés contre l'α-MSH présents chez les sujets sains et l'affinité des autoanticorps IgG dirigés contre l'α-MSH mesurée à l'étape a) est similaire à celle des autoanticorps IgG dirigés contre l'α-MSH présents chez les sujets sains.

## Patentansprüche

1. Ein Verfahren zur Diagnose von Anorexia nervosa des restriktiven Typus und Anorexia nervosa des Purging-Typus, das Verfahren umfassend die Schritte bestehend aus:
i) Messen der Affinität von Antikörpern, hervorgegangen aus einer biologischen Probe eines Patienten, gegen alpha-MSH (« alpha-Melanocyten-stimulierendes Hormon ») (α-MSH);
ii) Vergleichen des erhaltenen Affinitätswertes mit einem Referenz-Affinitätswert von Autoantikörpern gegen α-MSH bei einem gesunden Subjekt, und
Folgern, dass der Patient an Anorexia nervosa des Purging-Typus oder Anorexia nervosa des restriktiven Typus leidet, wenn die Affinität der Autoantikörper IgM gegen α-MSH gemessen in Schritt a) signifikant geringer als die der Autoantikörper IgM gegen α-MSH, die bei gesunden Subjekten vorhanden sind, ist, oder
der Patient eine Anorexia nervosa des Purging-Typus zeigt, wenn die Affinität der Autoantikörper IgG gegen α-MSH gemessen in Schritt a) signifikant höher gegenüber dem Referenz-Affinitätswert von Autoantikörpern IgG gegen α-MSH bei einem gesunden Subjekt ist, oder
der Patient eine Anorexia nervosa des restriktiven Typus zeigt, wenn die Affinität der Autoantikörper IgM gegen α-MSH gemessen in Schritt a) signifikant geringer als die der Autoantikörper IgM gegen α-MSH, die bei gesunden Subjekten vorhanden sind, ist, und die Affinität der Autoantikörper IgG gegen α-MSH gemessen in Schritt a) ähnlich der der Autoantikörper IgG gegen α-MSH, die bei gesunden Subjekten vorhanden sind, ist.

## Claims

1. A method for diagnosing restrictive-type anorexia nervosa and mixed-type anorexia nervosa with bulimia, said method comprising the steps consisting of:
i) measuring the affinity of antibodies originating from a biological sample from a patient which are directed against alpha-MSH ("alpha-melanocyte-stimulating hormone") (α-MSH);
ii) comparing the affinity value obtained with a reference affinity value for autoantibodies which are directed against α-MSH in a healthy subject, and
concluding that the patient is suffering from mixed-type anorexia nervosa with bulimia or restrictive-type anorexia nervosa if the affinity of the IgM autoantibodies directed against α-MSH measured in step a) is significantly lower than that of the IgM autoantibodies directed against α-MSH which are present in healthy subjects, or
the patient is exhibiting mixed-type anorexia nervosa with bulimia if the affinity of the IgG autoantibodies directed against α-MSH measured in step a) is significantly increased relative to the reference value for the affinity of IgG autoantibodies directed against α-MSH in a healthy subject, or
the patient is exhibiting restrictive-type anorexia nervosa when the affinity of IgM autoantibodies directed against α-MSH measured in step a) is significantly lower than that of the IgM autoantibodies directed against α-MSH which are present in healthy subjects and the affinity of the IgG autoantibodies directed against α-MSH measured in step a) is similar to that of the IgG autoantibodies directed against α-MSH which are present in healthy subjects.
